# EUROPÄISCHE PATENTSCHRIFT

(11) **EP 0 529 455 B1**
(45) Veröffentlichungstag und Bekanntmachung des Hinweises auf die Patenterteilung: **28.04.1999**
(21) Anmeldenummer: 92113978.8
(22) Anmeldetag: 17.08.1992
(51) Int. Cl.: G01N 33/36, D06B 23/00, D06B 23/24

(54) **Verfahren und Vorrichtung zum kontinuierlichen Messen von ionischen und nicht-ionischen Stoffen in laufendem Textilgut**
Method and device for continuously measuring ionic and non-ionic products in moving textiles
Procédé et dispositif pour la mesure continue de produits ioniques et non-ioniques dans un textile en défilement

(30) Priorität: 27.08.1991 DE 4128344; 23.09.1991 DE 4131616
(43) Veröffentlichungstag der Anmeldung: 03.03.1993
(73) Patentinhaber: Mahlo GmbH & Co. KG, 93342 Saal (DE)
(72) Erfinder: Beckstein, Hellmut, Dr.-Ing., W-8403 Bad Abbach (DE); Bors, Hans, Dipl.-Ing., CH-8117 Fällanden/Zürich (CH)
(74) Vertreter: Bohnenberger, Johannes, Dr.

(56) Entgegenhaltungen:
- EP-A- 0 005 443
- WO-A-88/02113
- DD-A- 68 888
- DD-A- 223 535
- DD-A- 249 930
- DE-A- 3 807 398
- GB-A- 1 268 492

## Beschreibung

Die Erfindung betrifft ein Verfahren zur Messung organischer und anorganischer Begleitfrachten von Textilgut bei Naßprozessen zur Behandlung des Textilguts sowie eine entsprechende Vorrichtung zur Durchführung des Verfahrens.

Ein wesentliches Problem bei Naßprozessen an laufendem Textilgut, z. B. beim Färben oder Hochveredeln, besteht darin, daß nach den vorausgehenden Prozessen Waschen, ggf. auch Absäuern, Abquetschen, Trocknen Fremdstoffe zurückbleiben können, die nachfolgende Behandlungsprozesse störend beeinflussen. Dies geschieht z. B. dann, wenn nicht genügend gewaschen bzw. nicht richtig in Kombination gewaschen und abgesäuert (bei vorausgehenden NaOH-Prozessen) wird. Andererseits will man den Waschvorgang aus Kostengründen minimieren. Nur als Beispiel sei hier angeführt, daß bei einer sauer geführten Hochveredelung (Vernetzung) im Textilgut verbleibende Restsäure überhöhte Effekte mit nicht tragbaren Festigkeitsverminderungen mit sich bringt, die Warenbahn wird also geschädigt. Ein zu hoher Gehalt an Elektrolyt oder Puffer bzw. eine ungünstige Kombination des Verhältnisses von Elektrolyt und Puffer führt zu einer Störung (bis zur völligen Inaktivierung) beim Färben, Bedrucken oder der Hochveredelung.

Aus der DD 223 535 A1 ist es bekannt, daß man die elektrische Leitfähigkeit einer Stoffbahn zwischen zwei Rollenelektroden messen kann, wobei die Leitfähigkeit ein Maß für die "Restchemikalien" in der Stoffbahn sein soll. Dies Verfahren ist aber nur bedingt anwendbar. Denn: trotz Waschens bestimmen nur einige der im Textilgut verbleibenden Begleitstoffe tatsächlich die Leitfähigkeit. Außerdem können selbst an und für sich leitende Substanzen an den Faseroberflächen adsorbiert und so für die Leitfähigkeitsmessung unzugänglich sein.

Gemäß der DD 249 930 B1 wird nicht die Leitfähigkeit an der Oberfläche der Stoffbahn, sondern vielmehr die Leitfähigkeit der (zuletzt) benutzten Behandlungsflüssigkeit, die ein Gemisch aus Behandlungsflüssigkeit und den aus der Stoffbahn ausgewaschenen Substanzen darstellt, gemessen. Auch hier werden somit nicht zur Leitfähigkeit beitragende Begleitstoffe nicht erfaßt.

In der DD-PS 68 888 wird vorgeschlagen, man solle eine chemische Analyse nicht der Wasch-Flüssigkeit durchführen, die beim "normalen" Abquetschen gewonnen werden kann, man solle vielmehr in einem gesonderten Probe-Abquetschvorgang nach dem (quantitativen) Abquetschen der Warenbahn abnehmbare Flüssigkeit analysieren, da deren Inhaltsstoffe denen im Inneren der Warenbahn genauer entsprechen. Dieses bekannte Verfahren ist insofern nachteilig, als bei starkem Abquetschen auf einen geringen Restfeuchtegehalt nur sehr schwer Proben durch ein weiteres, noch stärkeres Abquetschen entnehmbar sind, ggf. sogar das Textilgut geschädigt wird. Auch wird trotzdem nicht der innere ("Chemikalien-") Warenzustand erfaßt.

Aus der DE 38 07 398 A1 sind ein Verfahren und eine Vorrichtung zur kontinuierlichen Messung und Steuerung der Beladung von Textilgut mit wäßrigen, ionogenen Behandlungsmitteln bekannt. Dabei wird mit Hilfe von gegeneinander isoliert gelagerten Elektroden, die mit einer feuchten Textilgutbahn in Kontakt stehen, die Leitfähigkeit der Textilgutbahn zwischen den Elektroden gemessen.

Aus der GB-A-1-268-492 ist eine Testvorrichtung zur Bestimmung des Gehalts an Fettrückständen in einem Material bekannt. Dabei wird in eine Röhre Textilmaterial eingefüllt, welches zuvor mittels einer Waschlösung gereinigt wurde. Das Textilmaterial wird in der Röhre mit einem Kolben zusammengepreßt, wodurch Waschflüssigkeit aus dem Material herausgepreßt und in einem Behälter aufgefangen wird. Die Waschlösung wird wiederholt durch das in der Röhre vorhandene Material hindurchzirkuliert. Der Fettgehalt der herausgepreßten Waschlösung wird bestimmt, um so Rückschlüsse auf den Fettgehalt des Materials ziehen zu können. Durch diese Bestimmung des Fettgehalts soll die Zugabe von Waschzusätzen geregelt werden.

Der Erfindung liegt die Aufgabe zugrunde, Verfahren und Vorrichtung der eingangs genannten Art dahingehend weiterzubilden, daß in einfacher Weise in der abgequetschten Warenbahn enthaltene Begleitstoffe feststellbar sind.

Diese Aufgabe wird durch die Merkmale des Anspruches 1 bzw. 8 gelöst.

Ein wesentlicher Punkt der Erfindung liegt darin, daß nicht die an der Oberfläche des Textilgutes, sondern vielmehr die in seinem Inneren, zwischen den Oberflächen oder adsorbiert enthaltene Begleitfracht untersucht wird. Dies kann nun entweder dadurch erfolgen, daß man - nach dem Abquetschen - aus dem Textilgut abschnittsweise Probemengen der Begleitfracht durch Aufbringen und Absammeln von (frischem) Lösungsmittel entnimmt und die im Lösungsmittel enthaltene Begleitfracht quantitativ und/oder qualitativ bestimmt. Wichtig ist hierbei, daß das Lösungsmittel insbesondere das Innere der Warenbahn "durchspült" und die dort im Rest-Wasser (bzw. einem anderen Lösungsmittel) gelösten, an das Textilmaterial gebundenen oder auch dispergierte Begleitstoffe zumindest in einem exakt reproduzierbaren Maß mitnimmt. Dadurch, daß frisches Lösungsmittel verwendet wird, kann man den "Mitnahmegrad" wesentlich erhöhen. Gleichermaßen ist es möglich, durch die Art bzw. die Mischung des Lösungsmittels eine gegenüber dem herkömmlichen Lösungsmittel in der Waschflotte erheblich gesteigerte Mitnahmewirkung zu erzielen.

Vorzugsweise wird eine definierte Menge Lösungsmittel auf eine definierte Menge des Textilgutes aufgebracht und von diesem abgesammelt. Entsprechend der Geschwindigkeit der zu untersuchenden Warenbahn werden also das Aufbringen ebenso wie das Absammeln des Lösungsmittels geregelt. Dadurch wird auch gleichzeitig die mittlere Zeitdauer geregelt bzw. konstant gehalten, während derer das Lösungsmittel im Textilgut verbleibt. Die Aufbringung erfolgt also im wesentlichen punkt- bzw. linienförmig, während das Absammeln über eine längere Transport-Wegstrecke erfolgt.

Je nach Art der zu untersuchenden Begleitfrachten kann es von Vorteil sein, wenn das Lösungsmittel dampfförmig aufgebracht wird. Die Aufbringung erfolgt hierbei (wie auch bei flüssiger Aufbringung) mehr oder weniger senkrecht zur Flächenebene des Textilgutes, so daß das Lösungsmittel durch dieses hindurchgefördert wird. Dadurch ist sichergestellt, daß die im Inneren des Textilgutes enthaltenen Begleitfrachten bzw. Teilmengen hiervon mitgenommen werden. Die Mitnahmewirkung kann weiterhin über die Temperatur des Lösungsmittels, den verwendeten Volumenstrom und den Absaug-Unterdruck eingestellt werden.

Alternativ ist es möglich, die Leitfähigkeit im Inneren des Textilgutes, also nicht an seinen Oberflächen, sondern zwischen diesen zu bestimmen. Hierzu eignen sich in das Textilgut eingestochene Elektroden, z.B. Nadelelektroden, die von der Oberfläche von Walzen oder Rädern hervorstehen.

Um nun auch die Begleitfrachten zu bestimmen, welche keinen (wesentlichen) Einfluß auf die Leitfähigkeit des Gewebes ausüben, gewinnt man vorteilhafterweise für diese Begleitfrachten experimentelle Werte und speichert sie in Abhängigkeit von der dazu gemessenen Leitfähigkeit. Es hat sich nämlich überraschenderweise gezeigt, daß dann, wenn bei ein und demselben Prozeß sowohl die Leitfähigkeit beeinflussende (insbesondere ionische) Begleitfrachten, als auch die Leitfähigkeit nicht beeinflussende Begleitfrachten (z.B. nicht-ionische oder solche mit ans Textilgut gebundenen Ionen) vorliegen, zwischen diesen beiden an sich meßtechnisch nicht gleichzeitig erfaßbaren Stoffen eine experimentell bestimmbare Konzentrations-Beziehung besteht. Diese Beziehung kann derart sein, daß äußerst geringe Leitwerte, die z.B. gemäß der Lehre der DD 223 535 Al gar nicht mehr beachtet würden, dennoch in signifikanter Weise auf untolerierbar hohe, nicht-ionische Begleitfrachten schließen lassen können.

Vorrichtungsmäßig wird die eingangs genannte Aufgabe dadurch gelöst, daß eine erste Einrichtung dicht beim Textilgut angebracht ist, über welche Lösungsmittel auf das Textilgut (bzw. auf die laufende Warenbahn) aufbringbar ist. Eine zweite Einrichtung ist vorgesehen, welche ebenfalls dicht beim Textilgut sitzt und das Lösungsmittel aus dem Textilgut wieder absammelt und der Analyseeinrichtung zuführt.

Vorteilhafterweise weist die erste Einrichtung zum Aufbringen des Lösungsmittels eine Heizeinrichtung auf, um das Lösungsmittel auf eine definierte Temperatur zu bringen bzw. um es zu verdampfen und es als Dampf dem Textilgut zuzuführen. Das Lösungsmittel wird vorteilhafterweise hinsichtlich seiner Parameter, insbesondere hinsichtlich des Aufbringungsdruckes, des Volumenstroms und/oder der Temperatur geregelt, und zwar vorzugsweise in Abhängigkeit von der Geschwindigkeit, mit welcher das Textilgut an den Einrichtungen vorbeigefördert wird.

Das Absammeln erfolgt vorzugsweise über eine längere Wegstrecke (in Förderrichtung des Textilgutes gesehen) als das Aufbringen, damit das zusätzlich aufgebrachte Lösungsmittel im wesentlichen vollständig entfernt wird und eine nicht vollständig zu vernachlässigende mittlere Verweildauer des Lösungsmittels im Textilgut gewährleistet ist.

Weitere wesentliche Merkmale ergeben sich aus den Unteansprüchen und der nachfolgenden Beschreibung bevorzugter Ausführungsformen, die anhand von Abbildungen näher erläutert werden. Hierbei zeigen:
- Fig. 1: eine Prinzipskizze einer ersten Ausführungsform der Erfindung,
- Fig. 2: eine Prinzipskizze einer zweiten Anordnung, und
- Fig. 3: einen Teilschnitt durch eine Alternative zur Verwendung in der Anordnung nach Fig.2.

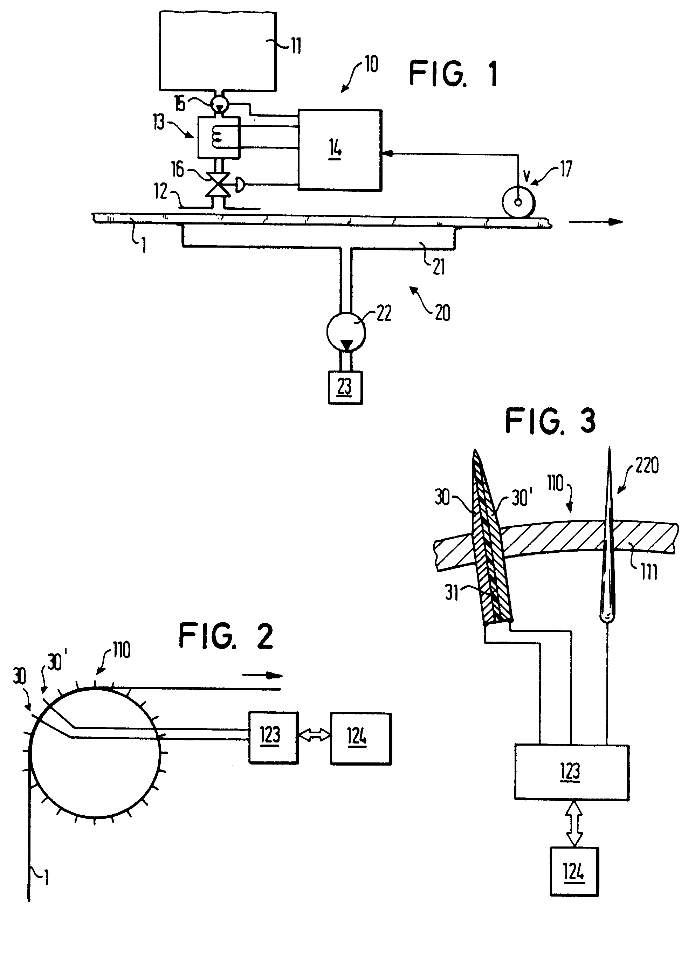

Bei der in Fig. 1 gezeigten Anordnung handelt es sich um eine solche, bei welcher Begleitfrachten durch Zugabe von Lösungsmittel aus dem Textilgut abgesammelt werden.

Das Textilgut 1 wird hierbei - kommend aus der Abquetscheinrichtung - einer Lösungsmittel-Zuführungseinrichtung 10 und einer dazugehörigen Lösungsmittel-Absammeleinrichtung 20 zugeführt.

Die Lösungsmittel-Zuführungseinrichtung 10 umfaßt einen Lösungsmittelbehälter 11, aus welchem Lösungsmittel, z.B. Wasser, mittels einer Lösungsmittelpumpe 15 abgepumpt, über eine Heizeinrichtung 13 erwärmt, durch ein einstellbares Ventil 16 hinsichtlich des Volumenstroms eingestellt und schließlich über eine Düse 12 in das Textilgut gedrückt wird. Die Düse 12 weist eine Breite von 0,5 mm - 10 mm, vorzugsweise 2 mm und eine Länge von 20 mm bis 300 mm, vorzugsweise 150 mm auf, welche vorzugsweise in horizontaler Richtung so angebracht wird, daß ein faltenfreier Gewebelauf garantiert ist.

Weiterhin ist ein Regler 14 vorgesehen, welcher die Pumpe 15, die Heizeinrichtung 13 und das Ventil 16 steuert. Die Steuerung erfolgt hierbei in Abhängigkeit der Geschwindigkeit v des Textilgutes 1, die beispielsweise mittels eines entsprechenden Drehmelders 17 abgetastet werden kann.

Auf der zur Düse 12 gegenüberliegenden Seite der Warenbahn 1 ist eine Ansaugdüse 21 angebracht, mittels derer das in das Textilgut 1 eingebrachte Lösungsmittel durch eine Pumpe 22 abgesammelt (ggf. dabei gleichzeitig kondensiert) und einer Analyseeinrichtung 23 zugeführt wird.

Die Absaugdüse hat eine wesentlich vergrößerte Öffnung (ca. 10 - 30%) und arbeitet bei einem Unterdruck von ca. 0 - 200 mbar, vorzugsweise bei 80 mbar. Wenn man dampfförmiges Lösungsmittel verwendet, so sind (in den Abbildungen nicht gezeigte) Kondensationseinrichtungen vorgesehen.

Die Analyseeinrichtung 23 enthält die gewünschten Sensoren, z. B. pH- oder ionensensitive Elektroden, Leitfähigkeits-Sensoren, Flammen-Photometer, Gaschromatographen u. a.

Das Lösungsmittel, das eine Mischung aus verschiedenen Lösungsmitteln und im allgemeinen auch Wasser enthält, wird bei Temperaturen von 25 °C bis 170 °C, vorzugsweise bei 120 °C sowie mit Volumenströmen zugeführt, welche das 2-50-fache, vorzugsweise das 5-10-fache der Gewebemasse beträgt, aus welcher die Begleitfrachten extrahiert werden.

Diese in Fig. 1 gezeigte Kombination aus Zuführungs- und Absammeleinrichtung für das Lösungsmittel kann nun fest relativ zum kontinuierlich geförderten Textilgut 1 oder aber in einer changierenden Bewegung quer zur Gewebelaufrichtung (siehe Pfeil in Fig. 1) bewegbar angeordnet sein. Durch eine solche Bewegbarkeit kann auch bei kleinbauender Ausführung eine Analyse über die gesamte Warenbahnbreite erfolgen.

Bei der in Fig. 2 gezeigten Anordnung werden Nadelelektroden 30, 30' in das Textilgut 1 eingestochen, die sich an einer umlaufenden Walze 110, über welche das Textilgut 1 geführt ist, befinden. Zwischen disen Nadeln 30, 30' wird nun mittels des Analysegerätes 123 der Widerstand und damit die Leitfähigkeit des Gewebes gemessen. Anhand dieser Leitfähigkeitswerte lassen sich bereits einige Begleitfrachten quantitativ bestimmen. Für solche Begleitfrachten, die keinen oder nur einen geringen Einfluß auf die Leitfähigkeit ausüben, wird zur Analyse ein Speicher 124 vorgesehen, in welchem experimentell gewonnene und anhand der Leitfähigkeit als Parameter gespeicherte Werte für diese anderen Begleitfrachten gespeichert sind.

Bei der in Fig. 3 gezeigten Anordnung sind die Elektroden 30, 30' zur Messung der Leitfähigkeit in einer einzigen Nadel unter Zwischenschaltung eines Dielektrikums 31 im Mantel 111 der Walze 110 untergebracht. Mit dieser Anordnung läßt sich somit bei entsprechender Dimensionierung auch die Leitfähigkeit im Inneren einer einzelnen Faser messen. Zusätzlich sind bei der in Fig. 3 gezeigten Ausführungsform nadelförmige ionensensitive Elektroden 220 im Mantel 111 der Walze 110 angebracht, so daß dadurch im Inneren des Textilgutes Konzentrationen von H-Ionen bzw. entsprechender anderer Ionen meßbar und mittels der Analyse-Einrichtung 123 analysierbar sind.

Vorzugsweise werden die beiden Verfahren bzw. die in den Fig. 1 und 2/3 gezeigten Vorrichtungen gleichzeitig verwendet, da die elektrische Leitfähigkeit im allgemeinen ohnehin gemessen werden muß.

Die nach dem erfindungsgemäßen Verfahren gewonnenen Meßwerte werden insbesondere zur Regelung der Waschprozesse oder zur Regelung von Neutralisationsprozessen in an sich bekannter Weise verwendet.

## Patentansprüche

1. Verfahren zur Messung organischer und anorganischer Begleitfrachten von Textilgut bei Naßprozessen zur Behandlung des Textilguts,
- wobei das Textilgut in Form einer zusammenhängenden Warenbahn vorliegt und
- die Messung mittels einer Analyseeinrichtung nach einem Abquetschen des Textilguts erfolgt,
- wobei auf das als zusammenhängende Warenbahn vorliegende Textilgut von einer Seite der Warenbahn her frisches Lösungsmittel aufgebracht wird,
- das nach Durchtritt durch die Warenbahn von der anderen Seite der Warenbahn her abgesammelt wird, derart,
- daß aus dem Inneren der Warenbahn abschnittsweise Probemengen der Begleitfracht erhalten werden,
- wobei die im Lösungsmittel enthaltene Begleitfracht quantitativ und/oder qualitativ bestimmt wird.

2. Verfahren nach Anspruch 1,
**dadurch gekennzeichnet,** daß
eine definierte Menge an Lösungsmittel auf eine definierte Menge des Textilguts aufgebracht und von diesem abgesammelt wird.

3. Verfahren nach einem der Ansprüche 1 oder 2,
**dadurch gekennzeichnet,** daß
das Lösungsmittel über eine definierte Zeitdauer im Textilgut belassen wird.

4. Verfahren nach einem der Ansprüche 1 bis 3,
**dadurch gekennzeichnet,** daß
das Lösungsmittel dampfförmig aufgebracht wird.

5. Verfahren nach einem der Ansprüche 1 bis 4,
**dadurch gekennzeichnet,** daß
das Lösungsmittel Wasser ist oder enthält und mit einer Temperatur von 25°C - 170°C, vorzugsweise 115 °C - 125 °C, aufgebracht wird.

6. Verfahren nach einem der Ansprüche 4 oder 5,
**dadurch gekennzeichnet,** daß
das Lösungsmittel in einem Volumenstrom aufgebracht wird, der das 2-50-fache, vorzugsweise das 5-10-fache der Gewebemasse beträgt, aus welcher extrahiert wird.

7. Verfahren nach einem der Ansprüche 1 bis 6,
**dadurch gekennzeichnet,** daß
das Lösungsmittel quer zur Flächenebene des Textilguts durch dieses hindurchgefördert wird.

8. Vorrichtung zur Durchführung des Verfahrens nach Anspruch 1,
**gekennzeichnet** durch
eine erste Einrichtung (10), die derart ausgebildet und dicht beim Textilgut (1) angebracht ist, daß frisches Lösungsmittel auf das Textilgut (1) aufbringbar ist, und durch eine zweite Einrichtung (20), die derart ausgebildet und dicht beim Textilgut (1) angebracht ist, daß das Lösungsmittel aus dem Textilgut abgesammelt und der Analyseeinrichtung (23) zuführbar ist.

9. Vorrichtung nach Anspruch 8,
**dadurch gekennzeichnet,** daß
die erste Einrichtung (10) eine Heizeinrichtung (13) zum Erwärmen und/oder Verdampfen des Lösungsmittels aufweist.

10. Vorrichtung nach einem der Ansprüche 8 oder 9,
**dadurch gekennzeichnet,** daß
die erste Einrichtung (10) Regeleinrichtungen (14) zum Regeln von Lösungsmittelparametern, insbesondere des Drucks und/oder Volumenstroms und/oder der Temperatur umfaßt.

11. Vorrichtung nach einem der Ansprüche 8 bis 10,
**dadurch gekennzeichnet,** daß
die zweite Einrichtung (20) eine derart ausgestaltete und relativ zu einer Zuführungsdüse (12) der ersten Einrichtung (10) angeordnete Absaugeinrichtung (21, 22) umfaßt, daß bei kontinuierlich gefördertem Textilgut (1) eine definierte mittlere Verweildauer des Lösungsmittels im Textilgut einstellbar ist.

## Claims

1. A method for measuring organic and inorganic foreign substances in textile material in wet processes for treating the textile material,
- the textile material being present in the form of a continuous material web and
- the measurement being effected by means of an analysis device following a squeezing of the textile material,
- fresh solvent being applied to the textile material present in the form of a continuous material web from one side of the material web,
- after passing through the material web, the solvent being collected from the other side of the material web in such a manner
- that sample quantities of the foreign substances are obtained from sections of the interior of the material web,
- the foreign substances contained in the solvent being determined quantitatively and/or qualitatively.

2. A method according to claim 1, characterised in that a defined quantity of solvent is applied to a defined quantity of the textile material and is collected therefrom.

3. A method according to one of claims 1 or 2,
characterised in that the solvent is left in the textile material for a defined period of time.

4. A method according to one of claims 1 to 3,
characterised in that the solvent is applied in the form of steam.

5. A method according to one of claims 1 to 4,
characterised in that the solvent is or contains water and is applied at a temperature of 25°C - 170°C, preferably 115°C - 125°C.

6. A method according to one of claims 4 or 5,
characterised in that the solvent is applied in a volume flow, which is 2 to 50 times, preferably 5 to 10 times the cloth mass from which it is extracted.

7. A method according to one of claims 1 to 6,
characterised in that the solvent is conveyed through the textile material transversely to the surface plane of the textile material.

8. A device for carrying out the method according to claim 1, characterised by a first device (10), which is constructed and fitted close to the textile material (1) in such a manner that fresh solvent can be applied to the textile material (1), and by a second device (20), which is constructed and fitted close to the textile material (1) in such a manner that the solvent can be collected from the textile material and can be supplied to the analysis device (23).

9. A device according to claim 8, characterised in that the first device (10) comprises a heating device (13) for heating and/or evaporating the solvent.

10. A device according to one of claims 8 or 9,
characterised in that the first device (10) comprises regulating devices (14) for controlling solvent parameters, more particularly the pressure and/or volume flow and/or the temperature.

11. A device according to one of claims 8 to 10,
characterised in that the second device (20) comprises a suction device (21, 22), which is constructed and displaceably arranged relative to a supply nozzle (12) of the first device (10) in such a manner that, with a continuously conveyed textile material (1), a defined average dwell time of the solvent in the textile material can be adjusted.

## Revendications

1. Procédé de mesure de substances organiques et inorganiques accompagnant un produit textile au cours d'un processus de traitement du produit textile par voie humide,
- dans lequel le produit textile se présente sous la forme d'un lé d'un seul tenant et
- la mesure s'effectue au moyen d'un dispositif d'analyse après essorage du produit textile,
- dans lequel un solvant frais est appliqué sur le produit textile, sous forme d'un lé d'un seul tenant, à partir d'une face du lé,
- le solvant est recueilli à partir de l'autre face du lé, après passage à travers ce demier,
- de manière à ce qu'on obtienne à partir de l'intérieur du lé, par endroits, des quantités-échantillons de la substance d'accompagnement,
- dans lequel la substance d'accompagnement contenue dans le solvant est déterminée quantitativement et/ou qualitativement.

2. Procédé selon la revendication 1, caractérisé en ce qu'une quantité déterminée de solvant est appliquée sur une quantité déterminée du produit textile et est recueillie à partir de celui-ci.

3. Procédé selon l'une des revendications 1 ou 2, caractérisé en ce que le solvant est laissé dans le produit textile pendant une durée déterminée.

4. Procédé selon l'une des revendications 1 à 3, caractérisé en ce que le solvant est appliqué sous forme vapeur.

5. Procédé selon l'une des revendications 1 à 4, caractérisé en ce que le solvant est ou contient de l'eau et est appliqué à une température comprise entre 25 °C et 170 °C, de préférence entre 115 °C et 125 °C.

6. Procédé selon l'une des revendications 4 ou 5, caractérisé en ce que le solvant est appliqué sous un courant en volume qui représente de 2 à 50 fois, de préférence de 5 à 10 fois la masse de tissu qui est soumise à extraction.

7. Procédé selon l'une des revendications 1 à 6, caractérisé en ce que le solvant est transporté à travers le produit textile, transversalement au plan de sa surface.

8. Dispositif pour la mise en oeuvre du procédé selon la revendication 1, caractérisé par un premier dispositif (10), qui est réalisé et monté tout près du produit textile (1) de manière à ce qu'un solvant frais puisse être appliqué sur le produit textile (1), ainsi que par un deuxième dispositif (20), qui est réalisé et monté tout près du produit textile (1) de manière à ce que le solvant soit recueilli du produit textile et puisse être envoyé au dispositif d'analyse (23).

9. Dispositif selon la revendication 8, caractérisé en ce que le premier dispositif (10) comporte un dispositif de chauffage (13) pour chauffer et/ou évaporer le solvant.

10. Dispositif selon l'une des revendications 8 ou 9, caractérisé en ce que le premier dispositif (10) comprend des dispositifs de régulation (14) pour la régulation des paramètres du solvant, en particulier de la pression et/ou du courant en volume et/ou de la température.

11. Dispositif selon l'une des revendications 8 à 10, caractérisé en ce que le deuxième dispositif (20) comprend un dispositif d'aspiration (21,22) conçu et disposé par rapport à une buse d'alimentation (12) du premier dispositif (10), de manière à ce que, lors du transport en continu du produit textile (1), on puisse régler une durée de séjour moyenne déterminée du solvant dans le produit textile.
